(19) Europäisches Patentamt
European Patent Office
Office européen des brevets

(11) Publication number: **0 159 619 A2**

(12) # EUROPEAN PATENT APPLICATION

(21) Application number: 85104334.9

(22) Date of filing: 10.04.85

(51) Int. Cl.⁴: **C 07 D 301/19**, B 01 J 31/34, C 07 F 5/04

(30) Priority: 20.04.84 IT 1249884

(43) Date of publication of application: 30.10.85 Bulletin 85/44

(84) Designated Contracting States: AT BE CH DE FR GB LI LU NL SE

(71) Applicant: MIRA LANZA S.p.a., 3, Piazza Brignole, I-16122 Genova (IT)

(72) Inventor: Tempesti, Ezio, 1, Viale Abruzzi, Milano (IT)
Inventor: Giuffrè, Luigi, 6, Via Passo di Fargorida, Milano (IT)
Inventor: Modica, Giovanni, 41, Via Archimede, Milano (IT)
Inventor: Montoneri, Enzo, 7, Via Bronzino, Milano (IT)

(74) Representative: Porsia, Attilio et al, c/o Succ. Ing. Fischetti & Weber Via Caffaro 3, I-16124 Genova (IT)

(54) **Process for the catalytic epoxidation of low molecular weight olefins, and catalyst for performing said process.**

(57) Catalytic epoxidation process of low molecular weight olefins, by means of a bimetallic boron-molybdenum reaction catalyst of the kind:

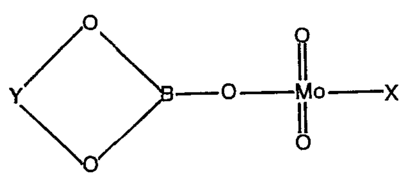

in which Y is a bivalent residue of an aromatic or aliphatic hydrocarbon; X is a generic ligand, and in which the oxygen bridge between B and Mo is of the chemical covalent type.

Advantageously, the said catalyst may be supported on polymeric matrices, for its use in heterogeneous phase.

"Process for the catalytic epoxidation of low molecular weight olefins, and catalyst for performing said process"

The present invention relates to the catalytic epoxidation of low molecular weight olefins.

It is known the direct oxidation of ethylene with air or oxygen, in presence of a silver-base catalyst, with weakly exothermic reaction toward 280°C:

$$CH_2{=}CH_2 + \tfrac{1}{2}\, O_2 \longrightarrow \underbrace{CH_2-CH_2-O}_{} \qquad (1)$$

The said process is the one which is almost exclusively used at the present time for the industrial

production of ethylene oxide.

For the production of propylene oxide by direct oxidation, at present two processes are generally used, namely the Halcon process and the Shell process.

The Halcon process (which is for example disclosed in the Belgian Patent No. 641452 and in the U.S.A. Patent No. 3,350,422, to which reference is made for more detailed information), operates in homogeneous phase, and it comprises generally two steps. In the first step a selfoxidation reaction of a suitable hydrocarbon, as for example ethylbenzene hydroperoxide is performed with molecular $O_2$, if necessary in presence of catalysts:

$$C_6H_5CH_2CH_3 + O_2 \longrightarrow C_6H_5CH(CH_3)O_2H \qquad (2)$$

In the second step the epoxidation reaction is performed under mild operational conditions preferably in presence of $Mo^{VI}$-base catalysts:

$$C_6H_5CH(CH_3)O_2H + CH_3CH\text{-}CH_2 \longrightarrow C_6H_5CH(CH_3)OH + \qquad (3)$$

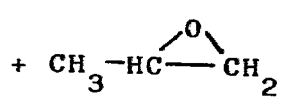

For conversion rates of ethylbenzene hydroperoxide in the range of 92%, propylene oxide selectivities in the range of 90% are claimed. The alcohol by-product is generally dehydrated to styrene

$$C_6H_5(CH_3)OH \xrightarrow{-H_2O} C_6H_5CH{=}CH_2 \qquad (4)$$

which may be sold or recycled after hydrogenation.

Among the advantages of the above process, the comparatively low cost of the oxidizing agent: the used hydroperoxides are less explosives and corrosives than the peracids used in the conventional processes. On the other side the economy of the process is conditioned by the price of the main by-product. Main disadvantage: recovery and partial loss of the catalyst from the reaction mass.

Shell (reference is made for example to the U.K. Patent No. 1,249,079 and to U.S. Patent No. 3,923,843) has developed a fixed bed catalyst which permits to work in continuous in heterogeneous phase thus obviating to the main drawback of the Halcon process. As far as the oxygen source is concerned, once again the hydroperoxides are used. On the contrary, as far as the catalyst is concerned, of which the total insolubility in the reaction mass is claimed, same is obtained by impregnation of silica with $TiCl_4$ or with an organic compound of Ti and subsequent calcination. It is believed that the active form of the catalyst contains $Ti^{IV}$ in tetrahedrical form chemically linked to SiO ligands:

(5)

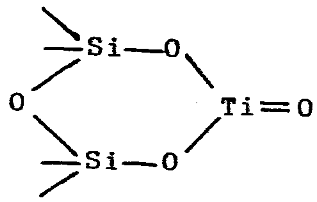

Said ligands should concur to increase the electrophil character of the $Ti^{IV}$ as well as to stabilize the reactive sites formed by the units Ti=O.

By the epoxidation of propylene with ethylbenzene hydroperoxide, selectivities in propylene oxide in the range of 93-94% with conversions of hydroperoxide in the range of 96% are claimed.

In addition to the above referred Halcon and Shell processes, many other processes have been proposed to epoxidate the olefinic double linkage. Some of the said processes are based on the use of boron derivates in epoxidation reactions. More particularly, it has been proposed to obtain:

1.- Propylene oxide from propylene and $H_2O_2$ in presence of $B_2O_3$ or of boric esters of the kind $(RO)_3B$ with convertition rates of 73% and selectivity rates of 43.9%;

2.- Different epoxides obtained from the corresponding olefins and hydroperoxides in presence of binary borides like WB, $LaB_6$, $TiB_2$ with very low or not referred selectivities;

3.- Different epoxides obtained from the corresponding olefins and perborates obtained by transesterification of boric esters with hydroperoxides.

Therefore, at least on the basis of what referred by the literature, there are no real alternatives with respect to what referred to for the Halcon and Shell processes.

It is therefore the object of the present invention a process and a catalyst for the catalytic epoxidation of low molecular weight olefins, by using peroxides as oxygen source.

It is a further object of the invention a catalytic epoxidation process of low molecular weight olefins, of the kind above referred, which works in heterogeneous phase.

By the term "low molecular weight olefins " as used in the following description, olefins are intended having as from 2 to 16 carbon atoms in the molecule.

It is known that it is possible to selectively functionalize polystyrenic resins by acid groups containing one boron atom. Polymers are obtained of the kind:

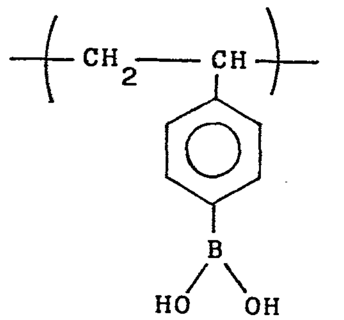

(6)

in which the substitution takes place only at para position and for which it is possible to vary at will the degree of functionalisation. The said polymers are at present advantageously used to remove or to chelate vicinal diols.

According to the invention it has been found that the functionalized polystyrenic resins permits the heterogeneization of a suitably selected complex of $Mo^{VI}$, as for example the $MoO_2$-(diacetylacetonate):

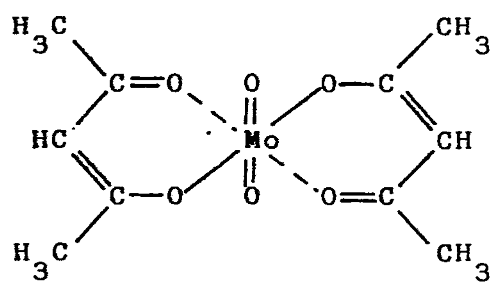

(7)

giving, through the removal of the acetylacetonic ligand by means of the boric acid group, $Mo^{VI}$ chemically linked to the boron through oxygen bridges:

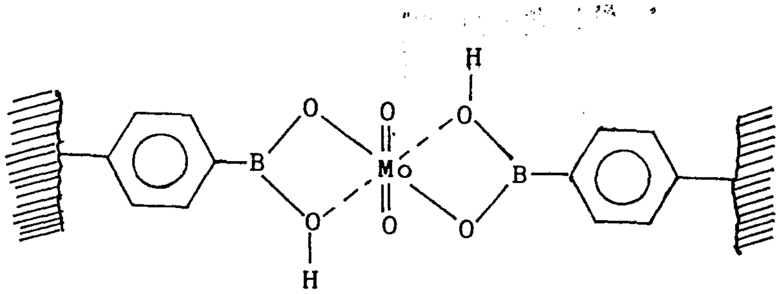

(8)

Therefore a mixed catalyst of the bimetallic type is obtained in which, it is proper to emphasize, the oxygen bridge between B and Mo is of the chemical covalent type. Therefore no solubilization of the catalyst in the reaction mass takes place.

This new kind of bimetallic catalyst has showed itself as particularly active for the epoxidation reaction of olefines by means of hydroperoxides.

In general, the new bimetallic catalysts B-Me according to the invention, in which Me is a metal selected from the group comprising Cr, Mo and W, and preferably is Mo, may be generally represented as follows:

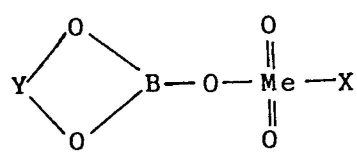

(9)

in which Me has the meaning explained above, Y is a bivalent residue of an aromatic hydrocarbon, or of an aliphatic hydrocarbon, and X is a generic ligand, and preferably a ligand which is present in the starting initial metal complex, the said bimetallic catalysts being further characterized by the presence of a covalent oxygen bridge link between B and Me.

For the synthesis of the above catalysts it is for example possible to start from the benzo-1,3 dioxa 2 borinic acid

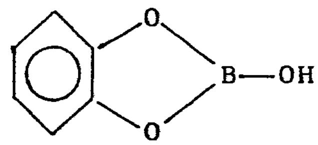

(10)

which in turn is obtained from pyrocatechin and $H_3BO_3$ previous azeotropic distillation of the esterification water. In order to link the Mo (preferably in the six oxidation form) to the B in a stable form and through oxygen bridges, it is possible for example to start from suitables $Mo^{VI}$ complexes which are available in commerce. In any case the formation of oxygen bridges between B and Mo is basically correlated to:

a)- the acid character of the functional group B-OH linked to the aromatic monomeric or polymeric nucleus;

b)- the character of the ligand which is present in the $Mo^{VI}$ complex used.

From a suitable combination of the above mentioned two working parameters it is possible to obtain very mild synthesis conditions for the said new catalysts. Thus for example, starting from the compound (10) and by employing the $MoO_2$-(diacetylacetonate) complex, the formation of oxygen bridges between boron and molybdenum takes place at environmental pressures and temperatures; more particularly by varying the starting molar ratio

between the compound (10) and the $Mo^{VI}$ complex it is possible to provide at will one or two acetylacetonics linkages which makes possible to vary at will the number of the oxygen bridges between boron and molybdenum.

On the other side in order to produce mixed catalysts containing B and Mo chemically linked through oxygen bridges it is not strictly necessary to start from aromatic type precursors containing acid functional units of the kind B-OH.

In order to ascertain this aspect, catalysts have been syntetized starting for example from an acid nonaromatic precursor of the kind

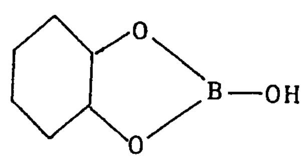

(11)

which was in turn obtained from mixtures of (cis and trans-) 1,2 - cyclohexanediol and $H_3BO_3$ after azeotropic distillation of the esterification water.

Once again, in order to subsequently link the Mo (preferably in the six oxidation form) in a stable manner through oxygen bridges it is possible to use anorganic compounds such as $MoO_2Cl_2$; again in this case the formation of bridges Mo-O-B with concurrent elimination of hydrochloric acid will be a function of:

a)- The acid character of the functional group B-OH

which is bound to the alyphatic matrix;

b)- The character of the substituent which is present in anorganic $Mo^{VI}$ compound used.

On the basis of the above discussion it follows that the synthesis of the new bimetallic B-Mo catalysts according to the present invention may be advantageously effected independently from the character (aromatic or alyphatic) of the support, provided that:

a.- it be possible to functionalize the support with boronics $-B(OH)_2$ or borinics $>B-OH$ groups having enough acidity;

b.- the following linking of the molybdenum results in the formation of covalent linkages with oxygen bridges between boron and molybdenum.

Some practical examples of synthesis of catalists B-Mo according to the invention will be now described, being understood that the said examples are given by way of explanatory and not limiting examples.

Example 1

Synthesis of the catalyst

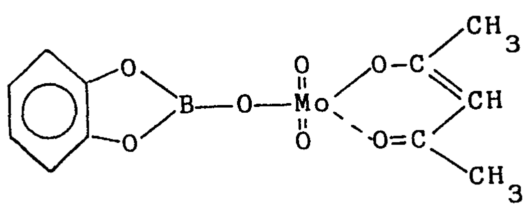

(12)

The synthesis of the above catalyst is performed in two steps.

A - First step

Synthesis of the benzo-1,3 dioxa 2 borinic acid referred to below:

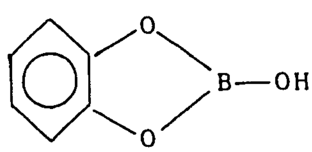

(13)

In a 250 ml flask, 3.0 g. of $H_3BO_3$ ($\sim$ 50 mmoles) and 5.5 g. of pyrocatechin ($\sim$ 50 mmoles) are introduced; 10 ml of xylene are added and the azeotropic distillation of the mixture xylene-water is performed.

The reaction is carried out up to completness:

a) by dosing with the Fischer method the outflowing water

(for each mole of $H_3BO_3$, two moles of water must outflow); b) by acidometric titration (via mannitol in excess) of the acidity which may be ascribed to the esterified boron.

B - Second step

At completion of the first step, the preparation of the proper catalyst is performed. For example, 10 ml of the xylenic solution obtained at the end of the first step, and containing 5.85 mmoles of benzo-1,3 dioxa 2-borinic acid are taken; to the said 10 ml, 1.90 g. of $MoO_2$(acetyl-acetonate)$_2$ (from the commerce: therefore we work with an equimolar ratio B/Mo) dissolved in 100 ml of chloroform are added at room temperature and in $N_2$ stream.

After having maintained over one night under stirring the reaction mass, the reaction solvent is distilled at room pressure. The thus obtained raw residue from the reaction mass is thereafter subjected to a purification cycle under hard vacuum, the said cycle comprising a first stripping at room temperature for 24 hours at $10^{-5}$/ $10^{-7}$ mm Hg and a second stripping for 24 hours at 115°C by the same vacuum conditions. The thus obtained product does not necessitate of further treatments and it is ready for use.

Example 2

Synthesis of the catalyst

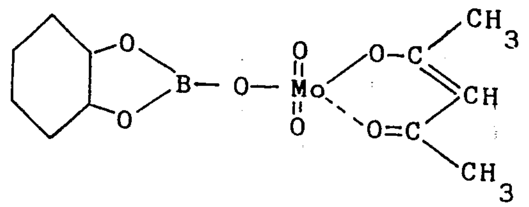

(14)

Substantially the same operations as referred in example 1 are performed however by providing the following changes:

A.- In the first step commercial grade (cis- and trans-) 1,2-cyclohexanediol is used instead of pyrocatechin.

From the operational point of view it is better to use the cis- isomer since esterification with boric acid is made easier.

B.- As an alternative to the cis- 1,2 cyclohexandiol, it is possible to start from the cis-1,2-cyclohexan-dimethanol which is easier to be found in commerce.
In the latter instance, when completed the preparation of the catalyst, the following product will be obtained:

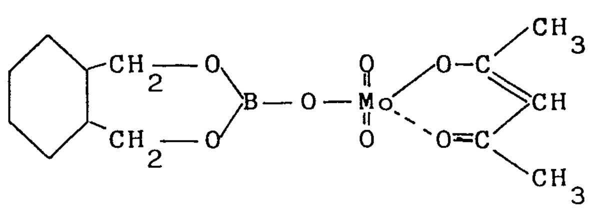

(15)

Example 3

As an alternative to what referred in the first step of Example 1, the benzo 1,3 dioxa 2 borinic acid may be

also obtained by following a method referred to in the literature (see Journal Chem. Soc., No. 1529 of 1959) according to which boron trichloride is used.

In the above instance the primary reaction product will be the following:

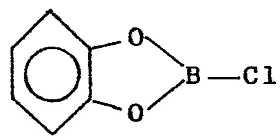

(16)

Through hydrolysis of the above product benzo 1,3 - dioxa 2 borinic acid is obtained.

In order to ascertain the activity of the B-Mo catalysts according to the invention, conventional hydroperoxides (obtained by non-catalytic self-oxidation with molecular $O_2$ of suitable hydrocarbons) have been used in absence and in presence of low molecular weight olefines.

The procedure of the tests performed is summarized as follows:

a) In absence of olefins

The evaluation of the activity has been made by comparing the decomposition rates of for example ethylbenzenhydroperoxide found in presence of the new B-Mo catalysts with respect to whose found with other catalytic systems assumed as reference.

By way of mere example, under the same testing conditions and by using the same hydroperoxide the activities of the three following different catalysts have been compared:

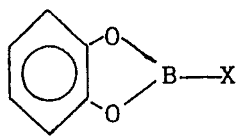

(17)

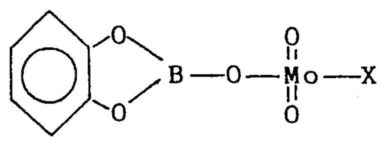

(18)

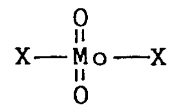

(19)

in which X = Acetylacetonic ligand; it is to be noted that the catalyst (19) is a classical catalyst in the Halcon process.

From the above comparisons it comes out that the new mixed B-Mo catalyst (18) clearly differs from that (17) containing only B; on the other side its behaviour with respect to ethylbenzenhydroperoxide may be related to that of the conventional catalyst (19)

b) In presence of olefins

In order to evaluate the activities of the mixed B-Mo catalysts with hydroperoxides in presence of olefins as for example propylene the same sperimental conditions normaly used in the Halcon process as far as temperature, pression, rest time, concentration of hydroperoxide, concentration of olefin, have been chosen. Once again for reference purposes conventional catalysts containing only Mo of use in the industry and non conventional catalysts containing only B, have been used. The sperimental results of the tests made, are referred next.

0159619

Test No. 1

Oxidation of $C_3H_6$ with Halcon conventional catalyst

$MoO_2$ ( acetylacetonate$)_2$ (20)

of the kind commercially available.

To carry out the above test, as well as to carry out the other comparative tests which are described later, a steel autoclave was used, having a free volume of about 300 ml, equipped with a 100 atm. manometer.

At room temperature, in the autoclave have been introduced:

a) - 14 mmoles of ethylbenzenhydroperoxide dissolved in 40 ml of distilled ethylbenzene

b) - 120 mg of $MoO_2$(acetylacetonate$)_2$ dissolved in 10 ml of distilled ethylbenzene

c) - 75 mmoles of propylene

- Thereafter the autoclave is pressurized at 42 atm with $N_2$ and same is plunged into a bath preheated at 120°C

| P. atm | T° C | t (minutes) |
|---|---|---|
| 41 | 120 | 0 |
| 43 | 120 | 10 |
| 47.5 | 122 | 20 |
| 49 | 122 | 30 |
| 49.8 | 120 | 40 |
| 50.5 | 120 | 50 |
| 51 | 124 | 60 |
| 51.2 | 125 | 70 |
| 51.2 | 125 | 90 |

- It is thereafter immediately cooled: the P is stabilized at 38 atm
- In the reaction mass:
  a) dosing of active oxygen: 2 mmoles. Conversion (consumed mmoles of hydroperoxide/mmoles of hydroperoxide at the start x 100) = 85%
  b) dosing of propylene oxide: 9.1 mmoles. Selectivity (mmoles propylene oxide/ consumed mmoles hydroperoxide x 100) = 75%

Test No. 2

Oxidation of $C_3H_6$ with a only boron base catalyst. For this test the following catalyst was used:

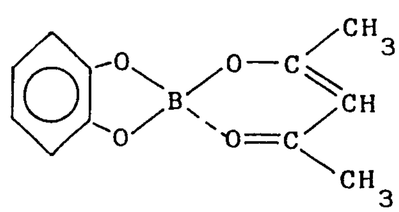

(21)

prepared as referred in literature (see Naturwissenschaften, 39, 280 (1952).

In the autoclave of Test 1, at room temperature have been introduced:

a) 21 mmoles of ethylbenzenhydroperoxide dissolved in 25 ml of distilled ethylbenzene

b) 160 mg of the catalyst containing only B shown by formula (21) dissolved in 25 ml of distilled ethylbenzene

c) 70 mmoles of propylene

- The autoclave is thereafter pressurized at 42 atm with $N_2$ and same is plunged into a bath preheated at 130°C

| P. atm | T°C | t(minutes) |
|---|---|---|
| 50.5 | 128 | 0 |
| 51.5 | 128 | 15 |
| 52.0 | 128 | 30 |
| 52.5 | 130 | 45 |
| 52.5 | 130 | 60 |
| 53.0 | 130 | 75 |
| 53.0 | 130 | 90 |
| 52.9 | 125 | 160 |
| 52.9 | 126 | 180 |

0159619

- It is thereafter immediately cooled: the P is stabilized at 36 atm

- In the reaction mass:

a) dosing of active oxygen: 8 mmoles. Conversion (mmoles consumed hydroperoxide/ mmoles hydroperoxide at the start x 100) = 61%

b) dosing of propylene oxide: 2 mmoles. Selectivity (mmoles propylene oxide/consumed mmoles hydroperoxide x 100) = 15%

<u>Test No. 3</u>

Oxidation of $C_3H_6$ with a B-Mo catalyst according to the invention.

For this Test the catalyst described in Example No. 1 was used:

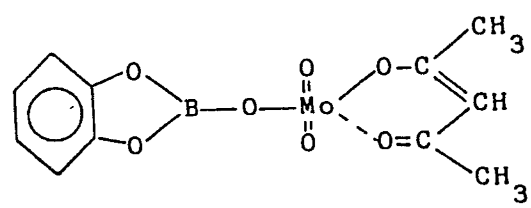

(22)

By operating in the autoclave according to the preceding Tests, at room temperature have been introduced:

a) 16 mmoles of ethylbenzenehydroperoxide dissolved in 20 ml of distilled ethylbenzene

b) 126 mg of the mixed B/Mo catalyst according to formula (22) dissolved in 30 ml of distilled ethylbenzene

c) 69 mmoles of propylene

- The autoclave is thereafter pressurized with $N_2$ at 43 atm and is plunged into a bath preheated at 120°C

| P. atm | T°C | t (minutes) |
|---|---|---|
| 42.2 | 118 | 0 |
| 46 | 118 | 10 |
| 46.5 | 121 | 20 |
| 46.5 | 123 | 30 |
| 46.9 | 120 | 40 |
| 47 | 123 | 50 |
| 47 | 123 | 60 |
| 47 | 123 | 70 |
| 47.1 | 123 | 80 |
| 47.1 | 123 | 90 |

- It is thereafter immediately cooled: the P is stabilized at 37 atm

- In the reaction mass:

a) dosing of active oxygen: 4 mmoles. Conversion (mmoles consumed hydroperoxide/ mmoles hydroperoxide at the start x 100) = 75%

b) dosing of propylene oxide: 8.5 mmoles. Selectivity (mmoles propylene oxide/ mmoles consumed hydroperoxide x 100)= 70%

On the attached drawings, in Figure 1 the decomposition curve of ethylbenzene hydroperoxide is shown in presence of a catalyst containing only boron (curve A) and in presence of a catalyst containing only Mo (curve B) as used in the Halcon process, and

in Figure 2 the decomposition curve of the same hydroperoxide in presence of the mixed B/Mo catalyst according to the invention is shown, at the temperature of 95°C (curve C) and at the temperature of 80°C (curve D).

In the diagrams of Figures 1 and 2, in abscissa the times (in prime minutes) and in ordinate the concentrations of mmoles/liter are shown.

From the results of the comparative Tests above disclosed, it appears evident that, in terms of selectivity in propylene oxide and of conversion with respect to the ethylbenzenehydroperoxide initially introduced, the mixed B-Mo catalysts which are the object of the present invention are by far better than those containing only B. With respect to those containing only Mo they have the great advantage that they may be supported on polymeric matrices and then, as in the Shell process, they may be used in heterogeneous phase.

Of course the present invention is not limited to the above referred examples, instead it comprises all those variants and modifications to a catalyst of the kind

referred to, falling within the limits of the appended claims.

0159619

## CLAIMS

1. Process for the catalytic epoxidation of alkenes and/or alkylated aromatic compounds, with at least one olefinic double linkage, having from 2 to 5 carbon atoms in the alkylic residue, characterized by the fact that as reaction catalyst a boron-metal bimetallic compound is used having the following general formula:

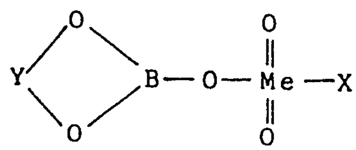

in which:
Y is a bivalent residue of an aromatic hydrocarbon, or of an aliphatic hydrocarbon
Me is a metal selected from the group comprising Cr, Mo, W and
X is a ligand.

2. The process according to claim 1, in which as oxidizing agents hydroperoxides are used.

3. The process according to claim 1, in which the said alkenes are olefins having from 2 to 16 carbon atoms in the molecule.

4. The process according to claim 1, in which the said alkylated aromatic compounds with at least one olefinic double linkage are alkenil-benzenic compunds having from

2 to 5 carbon atoms in the alkenilic residue.

5. The process according to claim 4, in which the said alkenil-benzenic compound is vinylbenzene.

6. The process according to claim 1, characterized by the fact that the catalytic epoxidation reaction of the said alkenes and/or of the said alkylated aromatic compounds with at least one olefinic double linkage takes place in heterogeneous phase.

7. A catalyst for the actuation of the process according to claim 1, characterized by the fact that it comprises a boron-metal bimetallic compound having the following general formula:

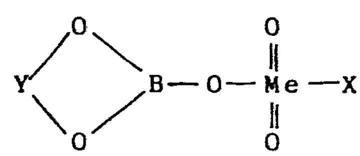

in which:

Y is a bivalent residue of an aromatic hydrocarbon, or of an alyphatic hydrocarbon Me is a metal selected from the group comprising Cr, Mo, W,

X is a ligand and in which the oxygen bridge between B and Me is of the chemical covalent type.

8. A catalyst according to claim 7, in which Me is molibdenum.

9. A catalyst according to claim 7, in which Y is a

bivalent residue of an aromatic hydrocarbon,

10. A catalyst according to claim 7, in which Y is a bivalent residue of an aliphatic hydrocarbon.

11. A catalyst according to claim 10, in which Y is a bivalent residue of a branched and cross-linked polymer.

12. A catalyst according to claim 7, in which X is a ligand which is present in the complex of the initial starting metal.

13. A catalyst according to claim 7, characterized by the fact that it is produced starting from an acid precursor of the kind:

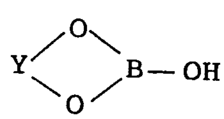

in which Y is a bivalent residue of an aromatic hydrocarbon, or of an aliphatic hydrocarbon, forming the support which may be functionalized with boronics-$B(OH)_2$ or borinics $>$B-OH groups having enough acidity.

14. A catalyst according to claim 13, in which the said acid precursor:

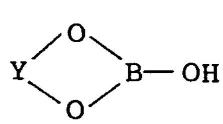

is reacted with an organic complex of a metal (Me) selected

from the group formed by Cr, Mo, W with oxidation number + 6, with formation of covalent linkages with oxygen bridges between boron and the said metal $(Me)^{VI}$.

15. A catalyst according to claim 13, in which the said acid precursor:

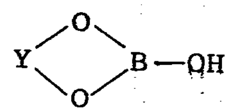

is reacted with an anorganic compound of a metal (Me) selected from the group of Cr, Mo, W, with oxidation number + 6, with formation of covalent linkages with oxygen bridges between boron and the said metal $(Me)^{VI}$.

16. A catalyst for the epoxidation of alkenes or of alkilated aromatic compounds, according to claim 7, comprising a boron molibdenum bimetallic compound of the kind:

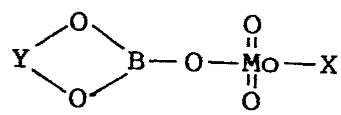

in which Y is an aromatic or aliphatic hydrocarbon, X is a ligand, and in which the oxygen bridge between B and Mo is of the chemical covalent type.

17. A catalyst according to claim 16, in which X is an acid precursor of the kind:

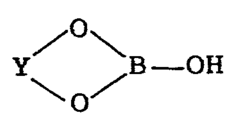

18. A catalyst according to claim 16, having the following formula:

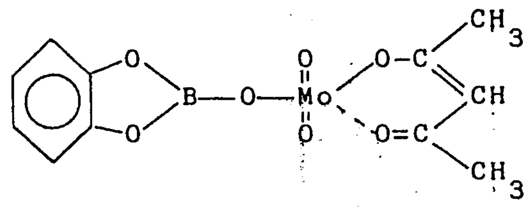

obtained as described in Example 1.

19. A catalyst according to claim 16, having the following formula:

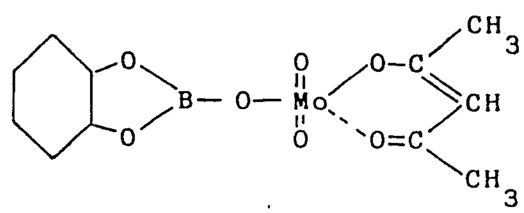

obtained as described in Example 2(A).

20. A catalyst according to claim 16, having the following formula:

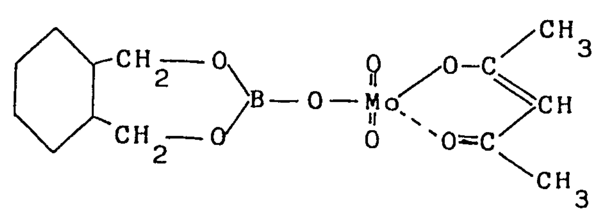

obtained as described in Example 2 (B).

21. A catalyst according to claim 7, characterized by the fact that it is supported on a polymeric matrix.

22. A catalyst according to claim 21, characterized by the fact that as polymeric matrix polystyrene is used.

Fig.1

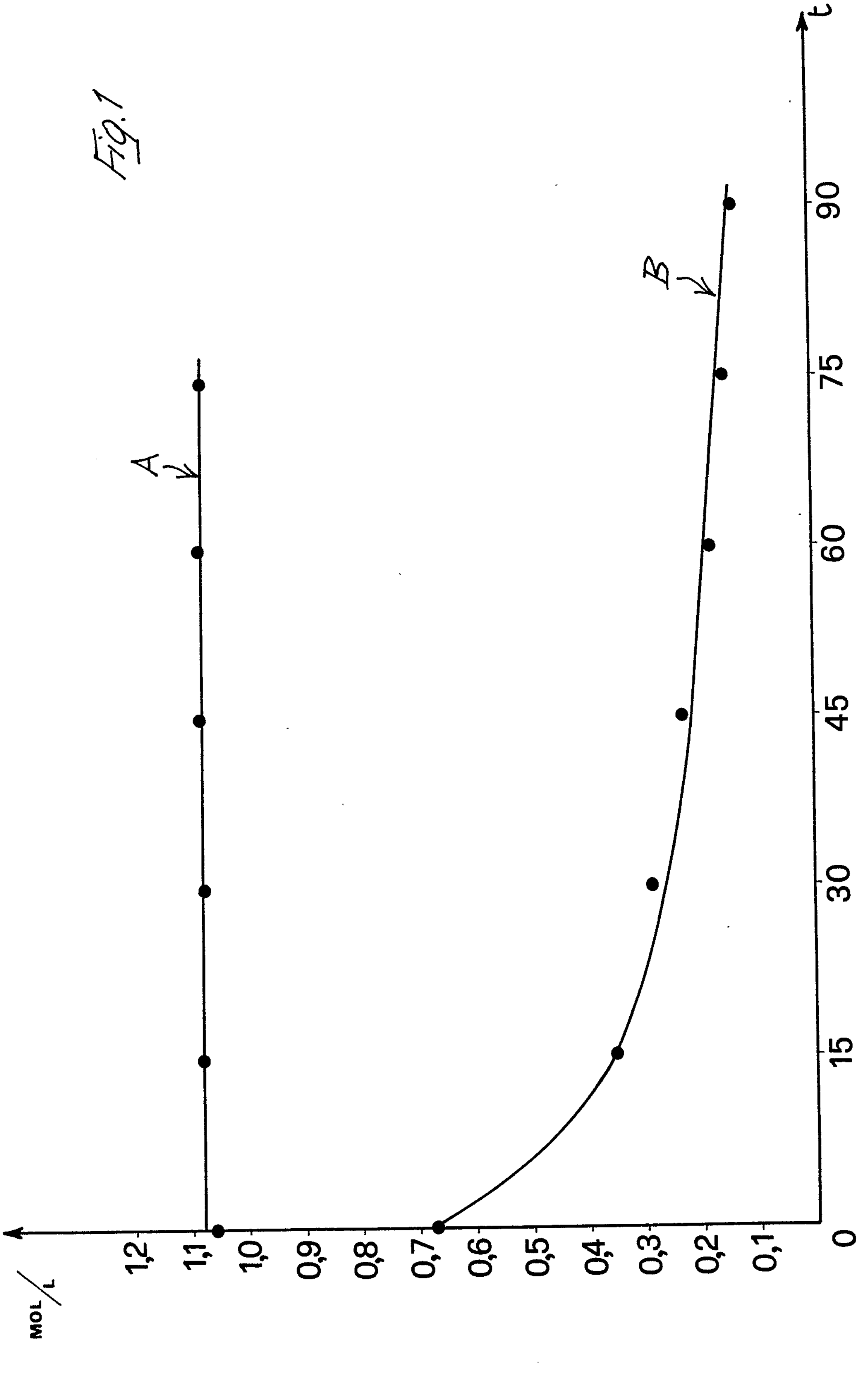

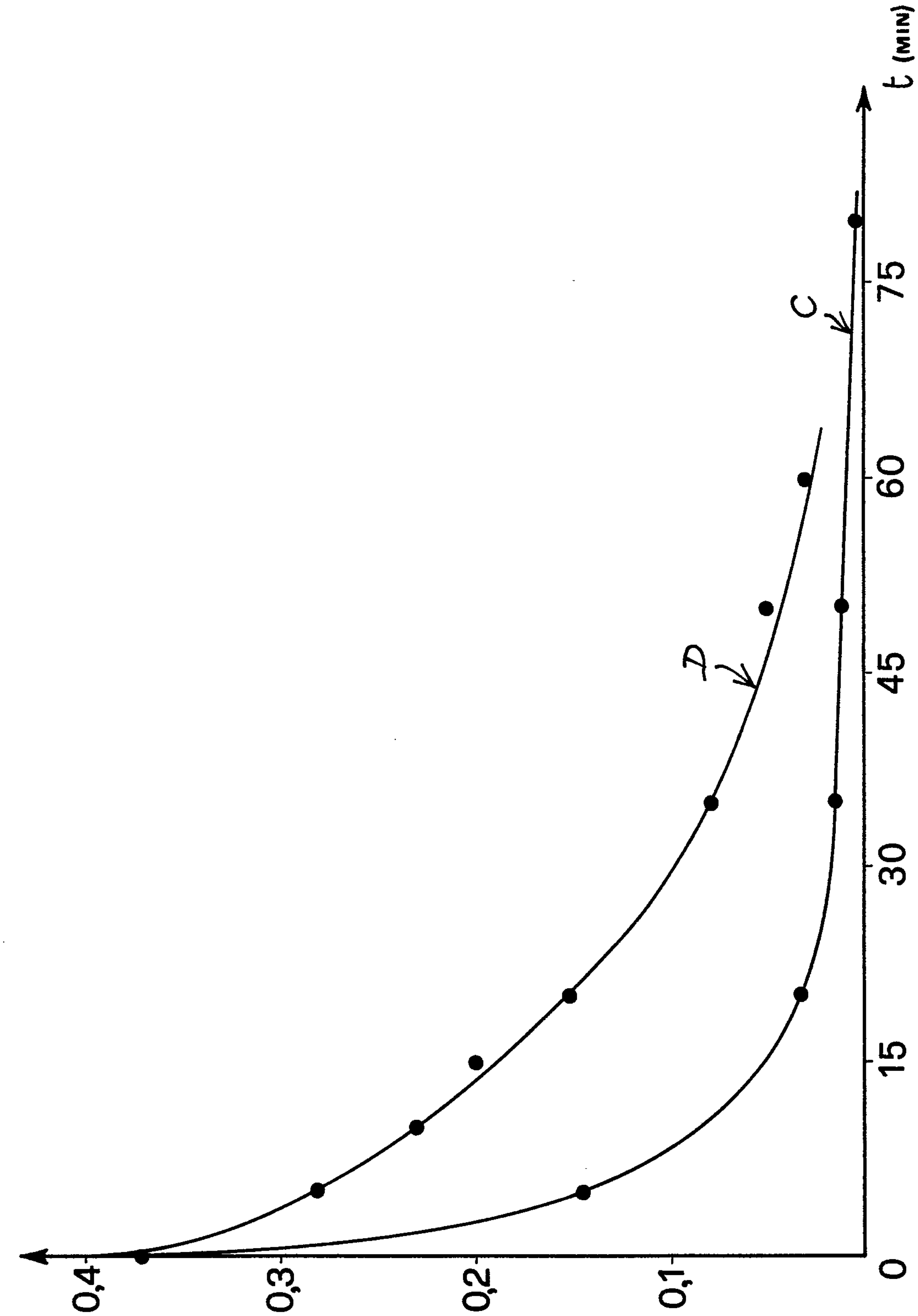


Fig. 2